# EUROPEAN PATENT SPECIFICATION

(11) **EP 1 320 377 B1**
(45) Date of publication and mention of the grant of the patent: **23.03.2011**
(21) Application number: 00961444.7
(22) Date of filing: 01.09.2000
(51) Int. Cl.: C07K 7/04, A61K 38/00

(54) **HLA BINDING PEPTIDES AND THEIR USES**
HLA-BINDUNGSPEPTIDE UND IHRE ANWENDUNGSZWECKE
PEPTIDES DE LIAISON AUX HLA ET LEURS UTILISATIONS

(43) Date of publication of application: 25.06.2003
(73) Proprietor: Epimmune Inc., San Diego, CA 92121 (US)
(72) Inventor: SETTE, Alessandro, La Jolla, CA 92037 (US); SIDNEY, John, La Jolla, CA 92037 (US); SOUTHWOOD, Scott, Santee, CA 92071 (US)
(74) Representative: Koefoed, Peter
(86) International application number: PCT/US2000/023913
(87) International publication number: WO 2002/020035

(56) References cited:
- WO-A-01/21189
- WO-A-93/25575
- WO-A-95/12677
- WO-A-99/58658
- DOOLAN DENISE L ET AL: "Degenerate cytotoxic T cell epitopes from P. falciparum restricted by multiple HLA-A and HLA-B supertype alleles" IMMUNITY, vol. 7, no. 1, 1997, pages 97-112, XP002360060 ISSN: 1074-7613
- SIDNEY J ET AL: "Practical, biochemical and evolutionary implications of the discovery of HLA class I supermotifs." IMMUNOLOGY TODAY. JUN 1996, vol. 17, no. 6, June 1996 (1996-06), pages 261-266, XP004034609 ISSN: 0167-5699
- SETTE A ET AL: "HLA supertypes and supermotifs: a functional perspective on HLA polymorphism" CURRENT OPINION IN IMMUNOLOGY, CURRENT BIOLOGY LTD, vol. 10, no. 4, August 1998 (1998-08), pages 478-482, XP004327132 ISSN: 0952-7915
- RAMMENSEE H-G ET AL: "MHC LIGANDS AND PEPTIDE MOTIFS: FIRST LISTING" IMMUNOGENETICS, SPRINGER VERLAG, BERLIN, DE, vol. 41, no. 4, February 1995 (1995-02), pages 178-228, XP000673045 ISSN: 0093-7711
- GULUKOTA K ET AL: "Two complementary methods for predicting peptides binding major histocompatibility complex molecules" JOURNAL OF MOLECULAR BIOLOGY, LONDON, GB, vol. 267, no. 5, 18 April 1997 (1997-04-18), pages 1258-1267, XP004456017 ISSN: 0022-2836
- HARLOW ET AL.: 'Molecular cloning and in vitro expression of a cDNA clone for human cellular tumor antigen p53' MOLECULAR AND CELLULAR BIOLOGY vol. 5, no. 7, July 1985, pages 1601 - 1610, XP002940044
- BERTONI ET AL.: 'Human class I supertypes and CTL repertoires extend to chimpanzees' JOURNAL OF IMMUNOLOGY vol. 161, no. 8, 15 October 1998, pages 4447 - 4455, XP002940043

## Description

### BACKGROUND OF THE INVENTION

The present invention relates to compositions and methods for preventing, treating or diagnosing a number of pathological states such as viral diseases and cancers. In particular, it provides novel peptides capable of binding selected major histocompatibility complex (MHC) molecules and inducing an immune response.

MHC molecules are classified as either Class I or Class II molecules. Class II MHC molecules are expressed primarily on cells involved in initiating and sustaining immune responses, such as T lymphocytes, B lymphocytes, macrophages, etc. Class II MHC molecules are recognized by helper T lymphocytes and induce proliferation of helper T lymphocytes and amplification of the immune response to the particular immunogenic peptide that is displayed. Class I MHC molecules are expressed on almost all nucleated cells and are recognized by cytotoxic T lymphocytes (CTLs), which then destroy the antigen-bearing cells. CTLs are particularly important in tumor rejection and in fighting viral infections.

The CTL recognizes the antigen in the form of a peptide fragment bound to the MHC class I molecules rather than the intact foreign antigen itself. The antigen must normally be endogenously synthesized by the cell, and a portion of the protein antigen is degraded into small peptide fragments in the cytoplasm. Some of these small peptides translocate into a pre-Golgi compartment and interact with class I heavy chains to facilitate proper folding and association with the subunit β2 microglobulin. The peptide-MHC class I complex is then routed to the cell surface for expression and potential recognition by specific CTLs.

The MHC class I antigens are encoded by the HLA-A, B, and C loci. HLA-A and HLA-B antigens are expressed at the cell surface at approximately equal densities, whereas the expression of HLA-C is significantly lower (perhaps as much as 10-fold lower). Each of these loci have a number of alleles.

Specific motifs for several of the major HLA-A alleles (copending U.S. Patent Applications 08/159,339 and 08/205,7 13, referred to here as the copending applications) and HLA-B alleles have been described. Several authors (Melief, Eur. J. Immunol., 21:2963-2970 (1991); Bevan, et al., Nature 353:852-955 (1991)) have provided preliminary evidence that class I binding motifs can be applied to the identification of potential immunogenic peptides in animal models. Strategies for identification of peptides or peptide regions capable of interacting with multiple MHC alleles has been described in the literature.

Because human population groups, including racial and ethnic groups, have distinct patterns of distribution of HLA alleles it will be of value to identify motifs that describe peptides capable of binding more than one HLA allele, so as to achieve sufficient coverage of all population groups. The present invention addresses these and other needs.

### SUMMARY OF THE INVENTION

The present invention provides vaccine compositions comprising immunogenic peptides having binding motifs for HLA alleles. The immunogenic peptides are about 9 to 10 residues in length and consist of SEQ ID NO. 13. In particular, an advantage of the peptides of the invention is their ability to bind to two or more different HLA alleles.

The present invention defines positions within a motif enabling the selection of peptides that will bind efficiently to more than one HLA-A, HLA-B or HLA-C alleles. Epitopes possessing the motif of the immunogenic peptides have been identified on potential target antigens including hepatitis B core and surface antigens (HBVc, HBVs), hepatitis C antigens, Epstein-Barr virus antigens, human immunodeficiency type-1 virus (HIV1) Lassa virus, p53 CEA, and Her2/neu. Thus, the invention further provides immunogenic peptides comprising sequences of target antigens.

The peptides of the invention are useful in pharmaceutical compositions for both *in vivo* and *ex vivo* therapeutic and diagnostic applications.

### Definitions

The term "peptide" is used interchangeably with "oligopeptide" in the present specification to designate a series of residues, typically L-amino acids, connected one to the other typically by peptide bonds between the alpha-amino and carbonyl groups of adjacent amino acids. The oligopeptides of the invention are less than about 15 residues in length and usually consist of between about 8 and about 11 residues, preferably 9 or 10 residues.

An "immunogenic peptide" is a peptide which comprises an allele-specific motif such that the peptide will bind an MHC molecule and induce a CTL response. Immunogenic peptides of the invention are capable of binding to an appropriate HLA molecule and inducing a cytotoxic T cell response against the antigen from which the immunogenic peptide is derived.

A "conserved residue" is a conserved amino acid occupying a particular position in a peptide motif typically one where the MHC structure may provide a contact point with the immunogenic peptide. One to three, typically two, conserved residues within a peptide of defined length defines a motif for an immunogenic peptide. These residues are typically in close contact with the peptide binding groove, with their side chains buried in specific pockets of the groove itself.

The term "motif" refers to the pattern of residues in a peptide of defined length, usually about 8 to about 11 amino acids, which is recognized by a particular MHC allele. The peptide motifs are typically different for each human MHC allele.

The term "supermotif" refers to motifs that, when present in an immunogenic peptide, allow the peptide to bind more than one HLA antigen. The supermotif preferably is recognized by at least one HLA allele having a wide distribution in the human population, preferably recognized by at least two alleles, more preferably recognized by at least three alleles, and most preferably recognized by more than three alleles.

The phrases "isolated" or "biologically pure" refer to material which is substantially or essentially free from components which normally accompany it as found in its native state. Thus, the peptides of this invention do not contain materials normally associated with their *in situ* environment, e.g., MHC I molecules on antigen presenting cells. Even where a protein has been isolated to a homogenous or dominant band, there are trace contaminants in the range of 5-10% of native protein which co-purify with the desired protein. Isolated peptides of this invention do not contain such endogenous co-purified protein.

The term "residue" refers to an amino acid or amino acid mimetic incorporated in an oligopeptide by an amide bond or amide bond mimetic.

### BRIEF DESCRIPTION OF THE DRAWINGS

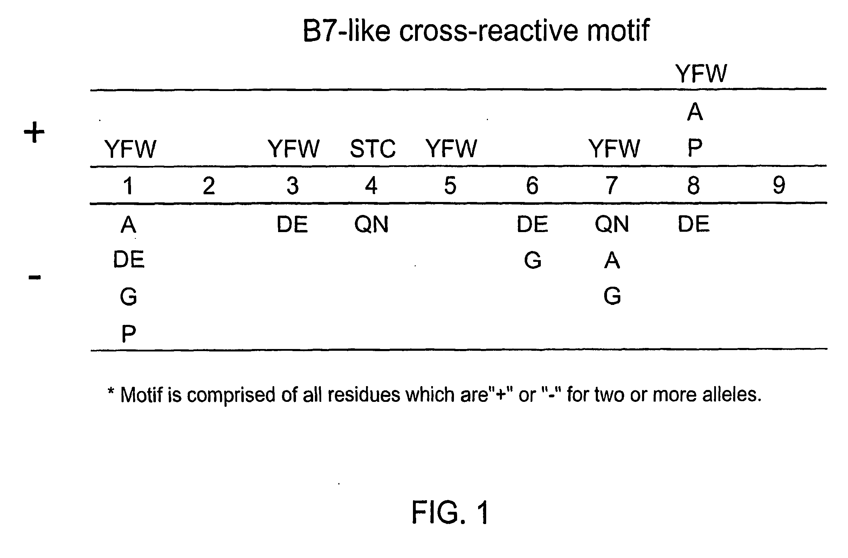

Figure 1 shows binding motifs for peptides capable of binding HLA alleles sharing the B7-like specificity.

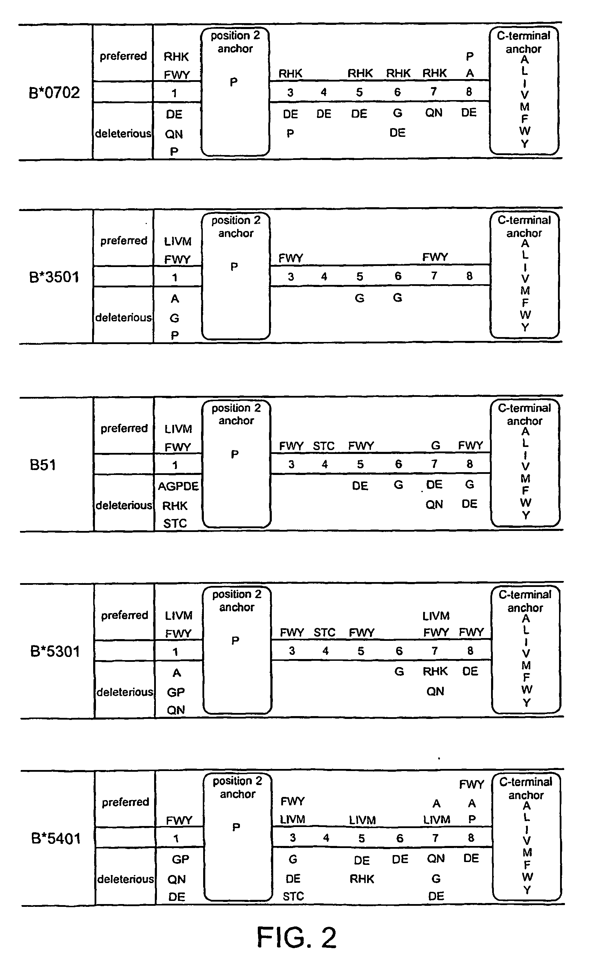

Figure 2 shows the B7-like cross-reactive motif.

### DESCRIPTION OF THE PREFERRED EMBODIMENTS

The present invention relates to the determination of allele-specific peptide motifs for human Class I MHC (sometimes referred to as HLA) allele subtypes. In particular, the invention provides motifs that are common to peptides bound by more than one HLA allele. By a combination of motif identification and MHC-peptide interaction studies, peptides useful for peptide vaccines have been identified.

Following the methods described in the copending applications noted above, certain peptides capable of binding at multiple HLA alleles which possess a common motif have been identified. The motifs of those peptides can be characterized as follows: N-XPXXXXXX(AVILM)-C; N-XPXXXXXXX(AVILM)-C; N-XPXXXXXX(FWY)-C; and N-XPXXXXXXX(FWY)-C. Motifs that are capable of binding at multiple alleles are referred to here as "supermotifs." The particular supermotifs above are specifically called "B7-like-supermotifs."

Immunogenic peptides of the invention are typically identified using a computer to scan the amino acid sequence of a desired antigen for the presence of the supermotifs. Examples of antigens include viral antigens and antigens associated with cancer. An antigen associated with cancer is an antigen, such as a melanoma antigen, that is characteristic of (i.e., expressed by) cells in a malignant tumor but not normally expressed by healthy cells. Examples of suitable antigens particularly include hepatitis B core and surface antigens (HBVc, HBVs) hepatitis C antigens, Epstein-Barr virus antigens, and human immunodeficiency virus (HIV) antigens, and also include prostate specific antigen (PSA), melanoma antigens (e.g., MAGE-1), human papilloma virus (HPV) antigens Lassa virus, p53 CEA, and Her2/neu; this list is not intended to exclude other sources of antigens.

Peptides comprising the supermotif sequences, including those found in proteins from potential antigenic sources are synthesized and then tested for their ability to bind to the appropriate MHC molecules in a variety of assays. The assays may use, for example, purified class I molecules and radioiodonated peptides. Alternatively, binding to cells expressing empty class I molecules can be detected by, for instance, immunofluorescent staining and flow microfluorimetry. Those peptides that bind to the class I molecule may be further evaluated for their ability to serve as targets for CTLs derived from infected or immunized individuals, as well as for their capacity to induce primary *in vitro or in vivo* CTL responses that can give rise to CTL populations capable of reacting with virally infected target cells or minor cells as therapeutic agents.

Recent evidence suggests however, that high affinity MHC binders might be, in most instances, immunogenic, suggesting that peptide epitopes might be selected on the basis of MHC binding alone.

Peptides comprising the supermotif sequences can be identified, as noted above, by screening potential antigenic sources. Useful peptides can also be identified by synthesizing peptides with systematic or random substitution of the variable residues in the supermotif, and testing them according to the assays provided. As demonstrated below, it is useful to refer to the sequences of the target HLA molecule, as well.

The nomenclature used to describe peptide compounds follows the conventional practice wherein the amino group is presented to the left (the N-terminus) and the carboxyl group to the right (the C-terminus) of each amino acid residue. In the formulae representing selected specific embodiments of the present invention, the amino-and carboxyl-terminal groups, although not specifically shown, are in the form they would assume at physiologic Ph values, unless otherwise specified. In the amino acid structure formulae, each residue is generally represented by standard three letter or single letter designations. The L-form of an amino acid residue is represented by a capital single letter or a capital first letter of a three-letter symbol, and the D-form for those amino acids having D-forms is represented by a lower case single letter or a lower case three letter symbol. Glycine has no asymmetric carbon atom and is simply referred to as "Gly" or G. The letter X in a motif represents any of the 20 amino acids found in Table 1, as well non-naturally occurring amino acids or amino acid mimetics. Brackets surrounding more than one amino acid indicates that the motif includes any one of the amino acids. For example, the supermotif "N-XPXXXXXX(AVYLM)-C" includes each of the following peptides: N-XPXXXXXXA-C, N-XPXXXXXXV-C, N-XPXXXXXXI-C, N-XPXXXXXXL-C, and N-XPXXXXXXM-C.

For peptide-based vaccines, the peptides of the present invention preferably comprise a motif which binds a number of HLA alleles which are well-represented in the population. Table 2 shows the distribution of certain HLA alleles in human populations.

**TABLE 1**

| | |
|---|---|
| Original Residue | Exemplary Substitution |
| Ala | Ser |
| Arg | Lys |
| Asn | Gln |
| Asp | Glu |
| Cys | Ser |
| Gln | Asn |
| Glu | Asp |
| Gly | Pro |
| His | Arg; Lys |
| Ile | Leu; Val; Met |
| Leu | Ile; Val; Met |
| Lys | Arg |
| Met | Leu; Ile; Val |
| Phe | Tyr; Trp |
| Ser | Thr |
| Thr | Ser |
| Trp | Tyr; Phe |
| Tyr | Trp; Phe |
| Val | Ile; Leu; Met |
| Pro | Gly |

**TABLE 2**

| Summary of Population Coverage by Currently Available Assays | | | | | | | |
|---|---|---|---|---|---|---|---|
| | | | | Phenotypic (Allelic) Frequency | | | |
| Antigen | HLA Allele | Cell Line(s) | Caucasian | Negro | Japanese | Chinese | Hispanic |
| A1 | A*0101 | Steinlin | 28.6 | 10.1 | 1.4 | 9.2 | 10.1 |
| A2.1 | A*0201 | JY | 45.8 | 30.3 | 42.4 | 54.0 | 43.0 |
| A3.2 | A*0301 | GM3107 | 20.6 | 16.3 | 1.2 | 7.1 | 14.8 |
| A11 | A*1101 | BVR | 9.9 | 3.8 | 19.7 | 33.1 | 7.3 |
| A24 | A*2401 | KT3 | 16.8 | 8.8 | 58.1 | 32.9 | 26.7 |
| All A | | | 88.9 | 59.8 | 91.6 | 94.6 | 80.2 |
| B7 | B*0701 | GM3107 | 17.7 | 15.5 | 9.6 | 6.9 | 11.8 |
| B8 | B*0801 | Steinlin | 18.1 | 6.3 | 0.0 | 3.6 | 9.0 |
| B27 | B*2705 | LG2 | 7.5 | 2.6 | 0.8 | 3.4 | 4.9 |
| B35 | B*3503 | BHM | 15.4 | 14.8 | 15.4 | 9.8 | 28.1 |
| B54 | B*5401 | KT3 | 0.0 | 0.0 | 12.4 | 8.6 | 0.0 |
| All B | | | 51.9 | 36.5 | 35.6 | 30.2 | 48.7 |
| Cw6 | Cw0601 | C1R | 17.6 | 13.7 | 2.2 | 19.0 | 12.2 |
| TOTAL | | | 95.7 | 76.5 | 94.7 | 96.6 | 91.0 |

For assays of peptide-HLA interactions (e.g., quantitative binding assays) cells with defined MHC molecules are useful. A large number of cells with defined MHC molecules, particularly MHC Class I molecules, are known and readily available. For example, human EBV-transfonned B cell lines have been shown to be excellent sources for the preparative isolation of class I and class II MHC molecules. Well-characterized cell lines are available from private and commercial sources, such as American Type Culture Collection ("Catalogue of Cell Lines and Hybridomas," 6th edition (1988) Rockville, Maryland, U.S.A.); National Institute of General Medical Sciences 1990/1991 Catalog of Cell Lines (NIGMS) Human Genetic Mutant Cell Repository, Camden, NJ; and ASHI Repository, Brigham and Women's Hospital, 75 Francis Street, Boston, MA 02115. Cell lines suitable as sources for various HLA-A alleles are described in the copending applications. Table 3 lists some B cell lines suitable for use as sources for HLA-B and HLA-C alleles, which are particularly useful in the present invention. All of these cell lines can be grown in large batches and are therefore useful for large scale production of MHC molecules. One of skill will recognize that these are merely exemplary cell lines and that many other cell sources can be employed.

**TABLE 3**

| HUMAN CELL LINES (HLA-B and HLA-C SOURCES) | |
|---|---|
| HLA-B allele | B cell line |
| B1801 | DVCAF |
| B3503 | EHM |
| B0701 | GM3107 |
| B1401 | LWAGS |
| B5101 | KAS116 |
| B5301 | AMAI |
| B0801 | MAT |
| B2705 | LG2 |
| B5401 | KT3 |
| B 1302 | CBUF |
| B4403 | PITOUT |
| B3502 | TISI |
| B3501 | BUR |
| B4001 | LB |
| HLA-C allele | B cell line |
| Cw0601 | C1R |

In the typical case, immunoprecipitation is used to isolate the desired allele. A number of protocols can be used, depending upon the specificity of the antibodies used. For example, allele-specific mAb reagents can be used for the affinity purification of the HLA-A, HLA-B, and HLA-C molecules. Monoclonal antibodies available for isolating various HLA molecules include those listed in Table 4. Affinity columns prepared with these mAbs using standard techniques are used to purify the respective HLA allele products.

**TABLE 4**

| ANTIBODY REAGENTS | |
|---|---|
| anti-HLA | Name |
| HLA-A2 | BB7.2 |
| HLA-A1 | 12/18 |
| HLA-A3 | GAPA3 (ATCC,HB122) |
| HLA-11,24.1 | A11.1M (ATCC, HB164) |
| HLA-A,B,C | W6/32 (ATCC, HB95) |
| monomorphic | B9.12.1 |
| HLA-B,C | B.1.23.2 |
| monomorphic | |

The capacity to bind MHC Class I molecules is measured in a variety of different ways. One means is a Class I molecular binding assay as described in Example 2, below. Other alternatives described in the literature include inhibition of antigen presentation (Sette, et al., J. Immunol. 141:3893 (1991)), *in vitro* assembly assays (Townsend, et al., Cell 62:285 (1990)) and FACS based assays using mutated cells, such as RMA.S (Melief, et al., Eur. J. Immunol. 21:2963 (1991)).

Next, peptides that test positive in the MHC class I binding assay are assayed for the ability of the peptides to induce specific CTL responses *in vitro.* For instance, antigen-presenting cells that have been incubated with a peptide can be assayed for the ability to induce CTL responses in responder cell populations. Antigen-presenting cells can be normal cells such as peripheral blood mononuclear cells or dendritic cells (Inaba, et al., J. Exp. Med. 166:182 (1987); Boog, Eur. I. Immunol. 18:219 (1988)). Alternatively transgenic mice comprising an appropriate HLA transgene can be used to assay the ability of a peptide to induce a response in cytotoxic T lymphocytes essentially as described in copending U.S. Patent Application No. 08/205,713.

Alternatively, mutant mammalian cell lines that are deficient in their ability to load class I molecules with internally processed peptides, such as the mouse cell lines RMA-S (Kärre, et al., Nature, 319:675 (1986); Ljunggren, et al., Eur. I. Immunol. 21:2963-2970 (1991)), and the human T cell hybridoma, T-2 (Cerundolo, et al., Nature 345:449-452 (1990)) and which have been transfected with the appropriate human class I genes are conveniently used, when peptide is added to them, to test for the capacity of the peptide to induce *in vitro* primary CTL responses. Other eukaryotic cell lines which could be used include various insect cell lines such as mosquito larvae (ATCC cell lines CCL 125, 126, 1660, 1591, 6585, 6586), silkworm (ATTC CRL 8851), armyworm (ATCC CRL 1711), moth (ATCC CCL 80) and Drosophila cell lines such as a Schneider cell line (see Schneider J. Embryo/. Exp. Morphol. 27:353-365 [1927]).

Peripheral blood lymphocytes are conveniently isolated following simple venipuncture or leukapheresis of normal donors or patients and used as the responder cell sources of CTL precursors. In one embodiment, the appropriate antigen-presenting cells are incubated with 10-100 µM of peptide in serum-free media for 4 hours under appropriate culture conditions. The peptide-loaded antigen-presenting cells are then incubated with the responder cell populations *in vitro* for 7 to 10 days under optimized culture conditions. Positive CTL activation can be determined by assaying the cultures for the presence of CTLs that kill radiolabeled target cells, both specific peptide-pulsed targets as well as target cells expressing endogenously processed form of the relevant virus or tumor antigen from which the peptide sequence was derived.

Specificity and MHC restriction of the CTL is determined by testing against different peptide target cells expressing appropriate or inappropriate human MHC class I. The peptides that test positive in the MHC binding assays and give rise to specific CTL responses are referred to herein as immunogenic peptides.

The immunogenic peptides can be prepared synthetically, or by recombinant DNA technology. Although the peptide will preferably be substantially free of other naturally occurring host cell proteins and fragments thereof, in some embodiments the peptides can be synthetically conjugated to native fragments or particles.

The polypeptides or peptides can be a variety of lengths, either in their neutral (uncharged) forms or in forms which are salts, and either free of modifications such as glycosylation, side chain oxidation, or phosphorylation or containing these modifications, subject to the condition that the modification not destroy the biological activity of the polypeptides as herein described.

Desirably, the peptide will be as small as possible while still maintaining substantially all of the biological activity of the large peptide. When possible, it may be desirable to optimize peptides of the invention to a length of 9 or 10 amino acid residues, commensurate in size with endogenously processed viral peptides or tumor cell peptides that are bound to MHC class I molecules on the cell surface.

Peptides having the desired activity may be modified as necessary to provide certain desired attributes, e.g., improved pharmacological characteristics, while increasing or at least retaining substantially all of the biological activity of the unmodified peptide to bind the desired MHC molecule and activate the appropriate T cell. For instance, the peptides may be subject to various changes, such as substitutions, either conservative or non-conservative, where such changes might provide for certain advantages in their use, such as improved MHC binding. By conservative substitutions is meant replacing an amino acid residue with another which is biologically and/or chemically similar, e.g., one hydrophobic residue for another, or one polar residue for another. The substitutions include combinations such as Gly, Ala; Val, Ile, Leu, Met; Asp, Glu; Asn, Gln; Ser, Thr; Lys, Arg; and Phe, Tyr. The effect of single amino acid substitutions may also be probed using D-amino acids. Such modifications may be made using well known peptide synthesis procedures, as described in e.g., Merrifield, Science 232:341-347 (1986), Barany and Merrifield, The Peptides, Gross and Meienhofer, eds. (N.Y., Academic Press), pp. 1-284 (1979); and Stewart and Young, Solid Phase Peptide Synthesis, (Rockford, III., Pierce), 2d Ed. (1984), incorporated by reference herein.

The peptides can also be modified by extending or decreasing the compound's amino acid sequence, e.g., by the addition or deletion of amino acids. The peptides or analogs of the invention can also be modified by altering the order or composition of certain residues, it being readily appreciated that certain amino acid residues essential for biological activity, e.g., those at critical contact sites or conserved residues, may generally not be altered without an adverse effect on biological activity. The non-critical amino acids need not be limited to those naturally occurring in proteins, such as L-α-amino acids, or their D-isomers, but may include non-protein amino acids as well, such as β-γ-δ-amino acids, as well as many derivatives of L-α-amino acids.

Typically, a series of peptides with single amino acid substitutions are employed to determine the effect of electrostatic charge, hydrophobicity, etc. on binding. For instance, a series of positively charged (e.g., Lys or Arg) or negatively charged (e.g., Glu) amino acid substitutions are made along the length of the peptide revealing different patterns of sensitivity towards various MHC molecules and T cell receptors. In addition, multiple substitutions using small, relatively neutral moieties such as Ala, Gly, Pro, or similar residues may be employed. The substitutions may be homo-oligomers or hetero-oligomers. The number and types of residues which are substituted or added depend on the spacing necessary between essential contact points and certain functional attributes which are sought (e.g., hydrophobicity versus hydrophilicity). Increased binding affinity for an MHC molecule or T cell receptor may also be achieved by such substitutions, compared to the affinity of the parent peptide. In any event, such substitutions should employ amino acid residues or other molecular fragments chosen to avoid, for example, steric and charge interference which might disrupt binding.

Amino acid substitutions are typically of single residues. Substitutions, deletions, insertions or any combination thereof may be combined to arrive at a final peptide. Substitutional variants are those in which at least one residue of a peptide has been removed and a different residue inserted in its place. Such substitutions generally are made in accordance with Table 1 when it is desired to finely modulate the characteristics of the peptide.

Substantial changes in function (e.g., affinity for MHC molecules or T cell receptors) are made by selecting substitutions that are less conservative than those in Table 1, i.e., selecting residues that differ more significantly in their effect on maintaining (a) the structure of the peptide backbone in the area of the substitution, for example as a sheet or helical conformation, (b) the charge or hydrophobicity of the molecule at the target site or (c) the bulk of the side chain. The substitutions which in general are expected to produce the greatest changes in peptide properties will be those in which (a) hydrophilic residue, e.g. seryl or threonyl, is substituted for (or by) a hydrophobic residue, e.g. leucyl, isoleucyl, phenylalanyl, valyl or alanyl; (b) a cysteine or proline is substituted for (or by) any other residue; (c) a residue having an electropositive side chain, e.g., lysl arginyl, or histidyl, is substituted for (or by) an electronegative residue e.g. glutamyl or aspartyl; or (d) a residue having a bulky side chain, e.g. phenylalanine, is substituted for (or by) one not having a side chain, e.g., glycine.

The peptides may also comprise isosteres of two or more residues in the immunogenic peptide. An isostere as defined here is a sequence of two or more residues that can be substituted for a second sequence because the steric conformation of the first sequence fits a binding site specific for the second sequence. The term specifically includes peptide backbone modifications well known to those skilled in the art. Such modifications include modifications of the amide nitrogen, the α-carbon, amide carbonyl, complete replacement of the amide bond, extensions, deletions or backbone crosslinks. See, generally, Spatola, Chemistry and Biochemistry of Amino Acids, Peptides and Proteins, Vol. VII (Weinstein ed., 1983).

Modifications of peptides with various amino acid mimetics or D-amino acids, for instance at the N- or C- termini, are particularly useful in increasing the stability of the peptide *in vivo.* Stability can be assayed in a number of ways. For instance, peptidases and various biological media, such as human plasma and serum, have been used to test stability. *See, e.g.,* Verhoef et al., Eur. J. Drug Metab. Pharmacokin. 11:291-302 (1986). Half life of the peptides of the present invention is conveniently determined using a 25% human serum (v/v) assay. The protocol is generally as follows. Pooled human serum (Type AB, non-heat inactivated) is delipidated by centrifugation before use. The serum is then diluted to 25% with RPMI tissue culture media and used to test peptide stability. At predetermined time intervals a small amount of reaction solution is removed and added to either 6% aqueous trichloracetic acid or ethanol. The cloudy reaction sample is cooled (4°C) for 15 minutes and then spun to pellet the precipitated serum proteins. The presence of the peptides is then determined by reversed-phase HPLC using stability-specific chromatography conditions.

The peptides of the present invention or analogs thereof which have CTL stimulating activity may be modified to provide desired attributes other than improved serum half life. For instance, the ability of the peptides to induce CTL activity can be enhanced by linkage to a sequence which contains at least one epitope that is capable of inducing a T helper cell response. Particularly preferred immunogenic peptides/T helper conjugates are linked by a spacer molecule. The spacer is typically comprised of relatively small, neutral molecules, such as amino acids or amino acid mimetics which are substantially uncharged under physiological conditions and may have linear or branched side chains. The spacers are typically selected from, e.g., Ala, Gly, or other neutral spacers of nonpolar amino acids or neutral polar amino acids. It will be understood that the optionally present spacer need not be comprised of the same residues and thus may be a hetero- or homo-oligomer. When present, the spacer will usually be at least one or two residues, more usually three to six residues. Alternatively, the CTL peptide may be linked to the T helper peptide without a spacer.

The immunogenic peptide may be linked to the T helper peptide either directly or via a spacer either at the amino or carboxy terminus of the CTL peptide. The amino terminus of either the immunogenic peptide or the T helper peptide may acylated. Exemplary T helper peptides include tetanus toxoid 830-843, influenza 307-319, malaria circumsporozoite 382-398 and 378-389.

In some embodiments it may be desirable to include in the pharmaceutical compositions of the invention at least one component which primes CTL. Lipids have been identified as agents capable of priming CTL *in vivo* against viral antigens. For example, palmitic acid residues can be attached to the alpha and epsilon amino groups of a Lys residue and then linked, e.g., via one or more linking residues such as Gly, Gly-Gly-, Ser, Ser-Ser, or the like, to an immunogenic peptide. The lipidated peptide can then be injected directly in a micellar form, incorporated into a liposome or emulsified in an adjuvant, e.g., incomplete Freund's adjuvant. In a preferred embodiment a particularly effective immunogen comprises palmitic acid attached to alpha and epsilon amino groups of Lys, which is attached via linkage, e.g., Ser-Ser, to the amino terminus of the immunogenic peptide.

As another example of lipid priming of CTL responses, *E*. *coli* lipoproteins, such as tripalmitoyl-S-glycerylcysteinlyseryl-serine (P₃CSS) I can be used to prime virus specific CTL when covalently attached to an appropriate peptide. See, Deres et al., Nature 342:561-564 (1989), incorporated herein by reference. Peptides of the invention can be coupled to P₃CSS, for example, and the lipopeptide administered to an individual to specifically prime a CTL response to the target antigen. Further, as the induction of neutralizing antibodies can also be primed with P₃CSS conjugated to a peptide which displays an appropriate epitope, the two compositions can be combined to more effectively elicit both humoral and cell-mediated responses to infection.

In addition, additional amino acids can be added to the termini of a peptide to provide for ease of linking peptides one to another, for coupling to a carrier support, or larger peptide, for modifying the physical or chemical properties of the peptide or oligopeptide, or the like. Amino acids such as tyrosine, cysteine, lysine, glutamic or aspartic acid, or the like, can be introduced at the C- or N-terminus of the peptide or oligopeptide. Modification at the C terminus in some cases may alter binding characteristics of the peptide. In addition, the peptide or oligopeptide sequences can differ from the natural sequence by being modified by terminal-NH₂ acylation, e.g., by alkanoyl (C₁-C₂₀) or thioglycolyl acetylation, terminal-carboxyl amidation, e.g., ammonia, methylamine, etc. In some instances these modifications may provide sites for linking to a support or other molecule.

The peptides of the invention can be prepared in a wide variety of ways. Because of their relatively short size, the peptides can be synthesized in solution or on a solid support in accordance with conventional techniques. Various automatic synthesizers are commercially available and can be used in accordance with known protocols. See, for example, Stewart and Young, Solid Phase Peptide Synthesis, 2d. ed., Pierce Chemical Co. (1984), *supra.*

Alternatively, recombinant DNA technology may be employed wherein a nucleotide sequence which encodes an immunogenic peptide of interest is inserted into an expression vector, transformed or transfected into an appropriate host cell and cultivated under conditions suitable for expression. These procedures are generally known in the art, as described generally in Sambrook et al., Molecular Cloning, A Laboratory Manual, Cold Spring Harbor Press, Cold Spring Harbor, New York (1982), which is incorporated herein by reference. Thus, fusion proteins which comprise one or more peptide sequences of the invention can be used to present the appropriate T cell epitope.

As the coding sequence for peptides of the length contemplated herein can be synthesized by chemical techniques, for example, the phosphotriester method of Matteucci et al., J. Am. Chem. Soc. 103:3185 (1981), modification can be made simply by substituting the appropriate base(s) for those encoding the native peptide sequence. The coding sequence can then be provided with appropriate linkers and ligated into expression vectors commonly available in the art, and the vectors used to transform suitable hosts to produce the desired fusion protein. A number of such vectors and suitable host systems are now available. For expression of the fusion proteins, the coding sequence will be provided with operably linked start and stop codons, promoter and terminator regions and usually a replication system to provide an expression vector for expression in the desired cellular host. For example, promoter sequences compatible with bacterial hosts are provided in plasmids containing convenient restriction sites for insertion of the desired coding sequence. The resulting expression vectors are transformed into suitable bacterial hosts. Of course, yeast or mammalian cell hosts may also be used, employing suitable vectors and control sequences.

The peptides of the present invention and pharmaceutical and vaccine compositions thereof are useful for administration to mammals, particularly humans, to treat and/or prevent viral infection and cancer. Examples of diseases which can be treated using the immunogenic peptides of the invention include prostate cancer, hepatitis B, hepatitis C, AIDS, renal carcinoma, cervical carcinoma, lymphoma, CMV and condlyloma acuminatum.

For pharmaceutical compositions, the immunogenic peptides of the invention are administered to an individual already suffering from cancer or infected with the virus of interest. Those in the incubation phase or the acute phase of infection can be treated with the immunogenic peptides separately or in conjunction with other treatments, as appropriate. In therapeutic applications, compositions are administered to a patient in an amount sufficient to elicit an effective CTL response to the virus or tumor antigen and to cure or at least partially arrest symptoms and/or complications. An amount adequate to accomplish this is defined as "therapeutically effective dose." Amounts effective for this use will depend on, e.g., the peptide composition, the manner of administration, the stage and severity of the disease being treated, the weight and general state of health of the patient, and the judgment of the prescribing physician, but generally range for the initial immunization (that is for therapeutic or prophylactic administration) from about 1.0 µg to about 5000 µg of peptide for a 70 kg patient, followed by boosting dosages of from about 1.0 µg to about 1000 µg of peptide pursuant to a boosting regimen over weeks to months depending upon the patient's response and condition by measuring specific CTL activity in the patient's blood. It must be kept in mind that the peptides and compositions of the present invention may generally be employed in serious disease states, that is, life-threatening or potentially life threatening situations. In such cases, in view of the minimization of extraneous substances and the relative nontoxic nature of the peptides, it is possible and may be felt desirable by the treating physician to administer substantial excesses of these peptide compositions.

For therapeutic use, administration should begin at the first sign of viral infection or the detection or surgical removal of tumors or shortly after diagnosis in the case of acute infection. This is followed by boosting doses until at least symptoms are substantially abated and for a period thereafter. In chronic infection, loading doses followed by boosting doses may be required.

Treatment of an infected individual with the compositions of the invention may hasten resolution of the infection in acutely infected individuals. For those individuals susceptible (or predisposed) to developing chronic infection the compositions are particularly useful in methods for preventing the evolution from acute to chronic infection. Where the susceptible individuals are identified prior to or during infection, for instance, as described herein, the composition can be targeted to them, minimizing need for administration to a larger population.

The peptide compositions can also be used for the treatment of chronic infection and to stimulate the immune system to eliminate virus-infected cells in carriers. It is important to provide an amount of immuno-potentiating peptide in a formulation and mode of administration sufficient to effectively stimulate a cytotoxic T cell response. Thus, for treatment of chronic infection, a representative dose is in the range of about 1.0 µg to about 5000 µg, preferably about 5 µg to 1000 µg for a 70 kg patient per dose. Immunizing doses followed by boosting doses at established intervals, e.g., from one to four weeks, may be required, possibly for a prolonged period of time to effectively immunize an individual. In the case of chronic infection, administration should continue until at least clinical symptoms or laboratory tests indicate that the viral infection has been eliminated or substantially abated and for a period thereafter.

The pharmaceutical compositions for therapeutic treatment are intended for parenteral, topical, oral or local administration. Preferably, the pharmaceutical compositions are administered parenterally, e.g., intravenously, subcutaneously, intradermally, or intramuscularly. Thus, the invention provides compositions for parenteral administration which comprise a solution of the immunogenic peptides dissolved or suspended in an acceptable carrier, preferably an aqueous carrier. A variety of aqueous carriers may be used e.g., water, buffered water, 0.4% saline, 0.3% glycine, hyaluronic acid and the like. These compositions may be sterilized by conventional, well known sterilization techniques, or may be sterile filtered. The resulting aqueous solutions may be packaged for use as is, or lyophilized, the lyophilized preparation being combined with a sterile solution prior to administration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc.

In some embodiments it may be desirable to include in the pharmaceutical composition at least one component which enhances priming of CTL. Lipids have been identified as agents capable of enhancing priming of CTL *in vivo* against viral antigens. For example, palmitic acid residues can be attached to the alpha and epsilon amino groups of a Lys residue and then linked, e.g. typically via one or more linking residues such as Gly, Gly-Gly-, Ser, Ser-Ser, or the like, to a synthetic peptide which comprises a class I-restricted CTL epitope. The lipidated peptide can be administered in saline or incorporated into a liposome emulsified in an adjuvant, e.g., incomplete Freund's adjuvant. In a preferred embodiment a particularly effective immunogen comprises palmitic acid attached to alpha and epsilon amino groups of Lys, which is attached via linkage, e.g., Ser-Ser, to the amino terminus of a class I restricted peptide having T cell determinants, such as those peptides described herein as well as other peptides which have been identified as having such determinants.

As another example of lipid priming of CTL responses, *E. coli* lipoprotein, such as tripalmitoyl-S-glycerylcysteinly-seryl-serine (P₃CSS), can be used to prime virus specific CTL when covalently attached to an appropriate peptide. See, Deres et al., Nature 342:561-564 (1989), incorporated herein by reference. Peptides of the invention can be coupled to P₃CSS, for example, and the lipopeptide administered to an individual to specifically prime a CTL. Further, as the induction of neutralizing antibodies can also be primed with P₃CSS conjugated to a peptide which displays an appropriate epitope, the two compositions can be combined to more effectively elicit both humoral and cell-mediated responses to viral infection.

The concentration of CTL stimulatory peptides of the invention in the pharmaceutical formulations can vary widely, i.e., from less than about 0.1%, usually at or at least about 2% to as much as 20% to 50% or more by weight, and will be selected primarily by fluid volumes, viscosities, etc., in accordance with the particular mode of administration selected.

The peptides of the invention may also be administered via liposomes, which serve to target the peptides to a particular tissue, such as lymphoid tissue, or targeted selectively to infected cells, as well as increase the half-life of the peptide composition. Liposomes include emulsions, foams, micelles, insoluble monolayers, liquid crystals, phospholipid dispersions, lamellar layers and the like. In these preparations the peptide to be delivered is incorporated as part of a liposome, alone or in conjunction with a molecule which binds to, e.g., a receptor prevalent among lymphoid cells, such as monoclonal antibodies which bind to the CD45 antigen, or with other therapeutic or immunogenic compositions. Thus, liposomes filled with a desired peptide of the invention can be directed to the site of lymphoid cells, where the liposomes then deliver the selected therapeutic/immunogenic peptide compositions. Liposomes for use in the invention are formed from standard vesicle-forming lipids, which generally include neutral and negatively charged phospholipids and a sterol, such as cholesterol. The selection of lipids is generally guided by consideration of, e.g., liposome size, acid lability and stability of the liposomes in the blood stream. A variety of methods are available for preparing liposomes, as described in, e.g., Szoka et al., Ann. Rev. Biophys. Bioeng. 9:467 (1980), U.S. Patent Nos. 4,235,871, 4,501,728, 4,837,028, and 5,019,369, incorporated herein by reference.

For targeting to the immune cells, a ligand to be incorporated into the liposome can include, e.g., antibodies or fragments thereof specific for cell surface determinants of the desired immune system cells. A liposome suspension containing a peptide may be administered intravenously, locally, topically, etc. in a dose which varies according to, *inter alia,* the manner of administration, the peptide being delivered, and the stage of the disease being treated.

For solid compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95% of active ingredient, that is, one or more peptides of the invention, and more preferably at a concentration of 25%-75%.

For aerosol administration, the immunogenic peptides are preferably supplied in finely divided form along with a surfactant and propellant. Typical percentages of peptides are 0.01%-20% by weight, preferably 1%-10%. The surfactant must, of course, be nontoxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. The surfactant may constitute 0.1%-20% by weight of the composition, preferably 0.25-5%. The balance of the composition is ordinarily propellant. A carrier can also be included, as desired, as with, e.g., lecithin for intranasal delivery.

In another aspect the present invention is directed to vaccines which contain as an active ingredient an immunogenically effective amount of an immunogenic peptide as described herein. The peptide(s) may be introduced into a host, including humans, linked to its own carrier or as a homopolymer or heteropolymer of active peptide units. Such a polymer has the advantage of increased immunological reaction and, where different peptides are used to make up the polymer, the additional ability to induce antibodies and/or CTLs that react with different antigenic determinants of the virus or tumor cells. Useful carriers are well known in the art, and include, e.g., thyroglobulin, albumins such as human serum albumin, tetanus toxoid, polyamino acids such as poly(lysine:glutamic acid), influenza, hepatitis B virus core protein, hepatitis B virus recombinant vaccine and the like. The vaccines can also contain a physiologically tolerable (acceptable) diluent such as water, phosphate buffered saline, or saline, and further typically include an adjuvant. Adjuvants such as incomplete Freund's adjuvant, aluminum phosphate, aluminum hydroxide, or alum are materials well known in the art. And, as mentioned above, CTL responses can be primed by conjugating peptides of the invention to lipids, such as P₃CSS. Upon immunization with a peptide composition as described herein, via injection, aerosol, oral, transdermal or other route, the immune system of the host responds to the vaccine by producing large amounts of CTLs specific for the desired antigen, and the host becomes at least partially immune to later infection, or resistant to developing chronic infection.

Vaccine compositions containing the peptides of the invention are administered to a patient susceptible to or otherwise at risk of viral infection or cancer to elicit an immune response against the antigen and thus enhance the patient's own immune response capabilities. Such an amount is defined to be an "immunogenically effective dose." In this use, the precise amounts again depend on the patient's state of health and weight, the mode of administration, the nature of the formulation, etc., but generally range from about 1.0 µg to about 5000 µg per 70 kilogram patient, more commonly from about 10 µg to about 500 µg mg per 70 kg of body weight.

In some instances it may be desirable to combine the peptide vaccines of the invention with vaccines which induce neutralizing antibody responses to the virus of interest, particularly to viral envelope antigens.

For therapeutic or immunization purposes, nucleic acids encoding one or more of the peptides of the invention can also be administered to the patient. A number of methods are conveniently used to deliver the nucleic acids to the patient. For instance, the nucleic acid can be delivered directly, as "naked DNA". This approach is described, for instance, in Wolff et. al., Science 247: 1465-1468 (1990) as well as U.S. Patent Nos. 5,580,859 and 5,589,466. The nucleic acids can also be administered using ballistic delivery as described, for instance, in U.S. Patent No. 5,204,253. Particles comprised solely of DNA can be administered. Alternatively, DNA can be adhered to particles, such as gold particles. The nucleic acids can also be delivered complexed to cationic compounds, such as cationic lipids. Lipid-mediated gene delivery methods are described, for instance, in WO 96/18372; WO 93/24640; Mannino and Gould-Fogerite (1988) BioTechniques 6(7): 682-691; Rose U.S. Pat No. 5,279,833; WO 91/06309; and Felgner et at. (1987) Proc. Natl. Acad. Sci. USA 84: 7413-7414. The peptides of the invention can also be expressed by attenuated viral hosts, such as vaccinia or fowlpox. This approach involves the use of vaccinia virus as a vector to express nucleotide sequences that encode the peptides of the invention. Upon introduction into an acutely or chronically infected host or into a noninfected host, the recombinant vaccinia virus expresses the immunogenic peptide, and thereby elicits a host CTL response. Vaccinia vectors and methods useful in immunization protocols are described in, e.g., U.S. Patent No. 4,722,848, incorporated herein by reference. Another vector is BCG (Bacille Calmette Guerin). BCG vectors are described in Stover et al. (Nature 351:456-460 (1991)) which is incorporated herein by reference. A wide variety of other vectors useful for therapeutic administration or immunization of the peptides of the invention, e.g., Salmonella typhi vectors and the like, will be apparent to those skilled in the art from the description herein.

A preferred means of administering nucleic acids encoding the peptides of the invention uses minigene constructs encoding multiple epitopes of the invention. To create a DNA sequence encoding the selected CTL epitopes (minigene) for expression in human cells, the amino acid sequences of the epitopes are reverse translated. A human codon usage table is used to guide the codon choice for each amino acid. These epitope-encoding DNA sequences are directly adjoined, creating a continuous polypeptide sequence. To optimize expression and/or immunogenicity, additional elements can be incorporated into the minigene design. Examples of amino acid sequence that could be reverse translated and included in the minigene sequence include: helper T lymphocyte epitopes, a leader (signal) sequence, and an endoplasmic reticulum retention signal. In addition, MHC presentation of CTL epitopes may be improved by including synthetic (e.g. poly-alanine) or naturally-occurring flanking sequences adjacent to the CTL epitopes.

The minigene sequence is converted to DNA by assembling oligonucleotides that encode the plus and minus strands of the minigene. Overlapping oligonucleotides (30-100 bases long) are synthesized, phosphorylated, purified and annealed under appropriate conditions using well known techniques. The ends of the oligonucleotides are joined using T4 DNA ligase. This synthetic minigene, encoding the CTL epitope polypeptide, can then be cloned into a desired expression vector.

Standard regulatory sequences well known to those of skill in the art are included in the vector to ensure expression in the target cells. Several vector elements are required: a promoter with a down-stream cloning site for minigene insertion; a polyadenylation signal for efficient transcription termination; an *E*. *coli* origin of replication; and an *E*. *coli* selectable marker (e.g. ampicillin or kanamycin resistance). Numerous promoters can be used for this purpose, *e.g*., the human cytomegalovirus (hCMV) promoter. *See,* U.S. Patent Nos. 5,580,859 and 5,589,466 for other suitable promoter sequences.

Additional vector modifications may be desired to optimize minigene expression and immunogenicity. In some cases, introns are required for efficient gene expression, and one or more synthetic or naturally-occurring introns could be incorporated into the transcribed region of the minigene. The inclusion of mRNA stabilization sequences can also be considered for increasing minigene expression. It has recently been proposed that immunostimulatory sequences (ISSs or CpGs) play a role in the immunogenicity of DNA vaccines. These sequences could be included in the vector, outside the minigene coding sequence, if found to enhance immunogenicity.

In some embodiments, a bicistronic expression vector, to allow production of the minigene-encoded epitopes and a second protein included to enhance or decrease immunogenicity can be used. Examples of proteins or polypeptides that could beneficially enhance the immune response if co-expressed include cytokines (e.g., 1L2, 1L12, GM-CSF), cytokine-inducing molecules (e.g. LeIF) or costimulatory molecules. Helper (HTL) epitopes could be joined to intracellular targeting signals and expressed separately from the CTL epitopes. This would allow direction of the HTL epitopes to a cell compartment different than the CTL epitopes. If required, this could facilitate more efficient entry of HTL epitopes into the MHC class II pathway, thereby improving CTL induction. In contrast to CTL induction, specifically decreasing the immune response by co-expression of immunosuppressive molecules (e.g. TGF-β) may be beneficial in certain diseases.

Once an expression vector is selected, the minigene is cloned into the polylinker region downstream of the promoter. This plasmid is transformed into an appropriate *E. coli* strain, and DNA is prepared using standard techniques. The orientation and DNA sequence of the minigene, as well as all other elements included in the vector, are confirmed using restriction mapping and DNA sequence analysis. Bacterial cells harboring the correct plasmid can be stored as a master cell bank and a working cell bank.

Therapeutic quantities of plasmid DNA are produced by fermentation in *E*. *coli*, followed by purification. Aliquots from the working cell bank are used to inoculate fermentation medium (such as Terrific Broth), and grown to saturation in shaker flasks or a bioreactor according to well known techniques. Plasmid DNA can be purified using standard bioseparation technologies such as solid phase anion-exchange resins supplied by Quiagen. If required, supercoiled DNA can be isolated from the open circular and linear forms using gel electrophoresis or other methods.

Purified plasmid DNA can be prepared for injection using a variety of formulations. The simplest of these is reconstitution of lyophilized DNA in sterile phosphate-buffer saline (PBS). A variety of methods have been described, and new techniques may become available. As noted above, nucleic acids are conveniently formulated with cationic lipids. In addition, glycolipids, fusogenic liposomes, peptides and compounds referred to collectively as protective, interactive, non-condensing (PINC) could also be complexed to purified plasmid DNA to influence variables such as stability, intramuscular dispersion, or trafficking to specific organs or cell types.

Target cell sensitization can be used as a functional assay for expression and MHC class I presentation of minigene-encoded CTL epitopes. The plasmid DNA is introduced into a mammalian cell line that is suitable as a target for standard CTL chromium release assays. The transfection method used will be dependent on the final formulation. Electroporation can be used for "naked" DNA, whereas cationic lipids allow direct *in vitro* transfection. A plasmid expressing green fluorescent protein (GFP) can be co-transfected to allow enrichment of transfected cells using fluorescence activated cell sorting (FACS). These cells are then chromium-51 labeled and used as target cells for epitope-specific CTL lines. Cytolysis, detected by 51Cr release, indicates production of MHC presentation of minigene-encoded CTL epitopes.

*In vivo* immunogenicity is a second approach for functional testing of minigene DNA formulations. Transgenic mice expressing appropriate human MHC molecules are immunized with the DNA product. The dose and route of administration are formulation dependent (e.g. IM for DNA in PBS, IP for lipid-complexed DNA). Twenty-one days after immunization, splenocytes are harvested and restimulated for 1 week in the presence of peptides encoding each epitope being tested. These effector cells (CTLs) are assayed for cytolysis of peptide-loaded, chromium-51 labeled target cells using standard techniques. Lysis of target cells sensitized by MHC loading of peptides corresponding to minigene-encoded epitopes demonstrates DNA vaccine function for *in vivo* induction of CTLs.

Antigenic peptides may be used to elicit CTL *ex vivo*, as well. The resulting CTL, can be used to treat chronic infections (viral or bacterial) or tumors in patients that do not respond to other conventional forms of therapy, or will not respond to a peptide vaccine approach of therapy. *Ex vivo* CTL responses to a particular pathogen (infectious agent or tumor antigen) are induced by incubating in tissue culture the patient's CTL precursor cells (CTLp) together with a source of antigen-presenting cells (APC) and the appropriate immunogenic peptide. After an appropriate incubation time (typically 1-4 weeks), in which the CTLp are activated and mature and expand into effector CTL, the cells are infused back into the patient, where they will destroy their specific target cell (an infected cell or a tumor cell).

The peptides may also find use as diagnostic reagents. For example, a peptide of the invention may be used to determine the susceptibility of a particular individual to a treatment regimen which employs the peptide or related peptides, and thus may be helpful in modifying an existing treatment protocol or in determining a prognosis for an affected individual. In addition, the peptides may also be used to predict which individuals will be at substantial risk for developing chronic infection.

The following example is offered by way of illustration, not by way of limitation.

### Example 1

### Identification of immunogenic peptides

Using the B7-like-supermotifs identified in the parent applications described above, sequences from a number of antigens were analyzed for the presence of the motifs. Tables 5-7 provide the results of these searches.

The above examples are provided to illustrate the invention but not to limit its scope. Other variants of the invention will be readily apparent to one of ordinary skill in the art and are encompassed by the appended claims. All publications, patents, and patent applications cited herein are hereby incorporated by reference.

**Table 5**

| Peptide | AA | Sequence | Source |
|---|---|---|---|
| 1 | 8 | VPLQLPPL | HIV1 REV73 |
| 2 | 8 | APTLWARM | HCV 2869 |
| 3 | 8 | IPFYGKAI | HCV 1378 |
| 4 | 8 | IPLVGAPL | HCV 137 |
| 5 | 8 | KPARLIVF | HCV 2608 |
| 6 | 8 | LPGCSFSI | HCV 169 |
| 7 | 8 | LPRRGPRL | HCV 37 |
| 8 | 8 | LPYIEQGM | HCV 1720 |
| 9 | 9 | CPKVSFEPI | HIV1 ENV 285 |
| 10 | 9 | IPIHYCAPA | HIV1 ENV 293 |
| 11 | 9 | HPVHAGPIA | HIV1 GAG 248 |
| 12 | 10 | HPRISSEVHI | HIV1 VIF 48 |
| 13 | 10 | LPINALSNSL | HCV |
| 14 | 11 | IPYNPQSQGVV | HIV1 POL 883 |
| 15 | 11 | APTLWARMILM | HCV 2869 |
| 16 | 9 | MPSLTLACL | Lassa np 179 |
| 17 | 9 | VPHVIEEMM | Lassa gp 11 |
| 18 | 10 | WPYIASRTSI | Lassa np 317 |
| 19 | 9 | FPVTPQVPL | HIV nef 84-92 analog |
| 20 | 9 | FPVRPQFPL | HIV nef 84-92 analog |
| 21 | 9 | IPIPSSWAF | HBV ENV 313 |
| 22 | 9 | FPIPSSWAF | HBV ENV 313 analog |
| 23 | 9 | IPITSSWAF | HBV ENV 313 analog |
| 24 | 9 | IPILSSWAF | HBV ENV 313 analog |
| 25 | 9 | FPHCLAFSL | HBV POL 541 analog |
| 26 | 9 | LPGCSFSIF | HCV Core 168 |
| 27 | 9 | FPGCSFSIF | HCV Core 168 analog |
| 28 | 9 | LPVCSFSIF | HCV Core 168 analog |
| 29 | 9 | LPGCSFSYF | HCV Core 168 analog |
| 30 | 9 | VPISHLYIL | MAGE2 170 |
| 31 | 9 | FPISHLYIL | MAGE2 170 analog |
| 32 | 9 | VPISHLYAL | MAGE2 170 analog |
| 33 | 9 | MPVAGLLII | MAGE3 196 analog |
| 34 | 9 | FPVRMQVPL | HIV nef 84-92 analog |
| 35 | 9 | IPIPMSWAF | HBV ENV 313 analog |
| 36 | 9 | FPHCLAFAL | HBV POL 541 analog |
| 37 | 9 | LPGCMFSIF | HCV Core 168 analog |
| 38 | 9 | VPISMLYIL | MAGE2 170 analog |
| 39 | 9 | FPVRPQVPL | HIV nef 84-92 |
| 40 | 9 | FPVTMFFAL | HIV nef 84-92 (a) |
| 41 | 9 | FPVTMFFAM | HIM nef 84-92 (a) |
| 42 | 9 | FPVRMFFAF | HN nef 84-92 (a) |
| 43 | 9 | FPVRMFFAL | HIV nef 84-92 (a) |
| 44 | 9 | FPVTFFFAL | HIV nef 84-92 (a) |
| 45 | 9 | FPVTMQFAF | HIV nef 84-92 (a) |
| 46 | 9 | FPVTMQFAL | HIV nef 84-92 (a) |
| 47 | 9 | FPVTMFSAF | HIV nef 84-92 (a) |
| 48 | 9 | FPVTMFSAL | HIV nef 84-92 (a) |
| 49 | 9 | FPVRPQVPA | HIV nef 84-92 (a) |
| 50 | 9 | FPVRPQVPV | HIV nef 84-92 (a) |
| 51 | 9 | FPVRPQVPI | HIV nef 84-92 (a) |
| 52 | 9 | FPVRPQVPM | HIV nef 84-92 (a) |
| 53 | 9 | FPVRPQVPF | HIV nef 84-92 (a) |
| 54 | 9 | FPVRPQVPW | HIV nef 84-92 (a) |
| 55 | 9 | FPVRPQVPH | HIV nef 84-92 (a) |

The peptides listed in Table 6 were identified as described above and are grouped according to pathogen or antigen from which they were derived.

**Table 6**

| HBV | | |
|---|---|---|
| **SEQ ID NO** | **Sequence** | **Source** |
| 56 | IPIPSSWAF | ENV.313 |
| 57 | HPAAMPHLL | POL.429 |
| 58 | FPHCLAFSYM | POL.530 |
| 59 | YPALMPLYA | POL.640 |
| 60 | LPVCAFSSA | X.58 |

| HCV | | |
|---|---|---|
| **SEQ ID NO** | **Sequence** | **Source** |
| 61 | LPGCSFSIF | CORE. 169 |

| HIV1 | | |
|---|---|---|
| **SEQ ID NO** | **Sequence** | **Source** |
| 62 | FPVRPQVPL | NEF.89 |
| 63 | YPLASLRSLF | GAG.552 |
| 64 | VPLQLPPL | REV.73 |

| Plasmodium falciparum | | |
|---|---|---|
| **SEQ ID NO** | **Sequence** | **Source** |
| 65 | TPYAGEPAPF | SSP2.539 |

| MAGE2/3 | | |
|---|---|---|
| **SEQ ID NO** | **Sequence** | **Source** |
| 66 | MPKAGLLII | MAGE3.196 |
| 67 | VPISHLYIL | MAGE2.170 |
| 68 | LPTTMNYPL | MAGE3.71 |

| Her2/neu | | |
|---|---|---|
| **SEQ ID NO** | **Sequence** | **Source** |
| 69 | LPQPPICTI | Her2/neu.941 |
| 70 | LPTNASLSF | Her2/neu.65 |
| 71 | MPNQAQMRI | Her2/neu.706 |

**Table 7 provides additional peptides identified using the methods described above.**

| Peptide | AA | Sequence | Antigen Protein or Molecule | | | | 1st Position | B*0702 |
|---|---|---|---|---|---|---|---|---|
| 1292.01 | 9 | SPRTLNAWI | HIV | GAG | 180 | 0.4200 | | |
| 1292.02 | 9 | KPCVKLTPI | HIV | ENV | 130 | 0.1100 | | |
| 1292.03 | 9 | SPAIFQSSI | HIV | POL | 335 | 0.3100 | | |
| 1292.07 | 10 | LPQGWKGSPI | HIV | POL | 328 | 0.0740 | | |
| 1292.13 | 9 | HPVHAGPIA | HIV | GAG | 248 | 0.1100 | | |
| 1292.14 | 9 | HPVHAGPII | HIV | GAG | 248 | 0.4100 | | |
| 1292.17 | 9 | PPVVHGCPL | HIV | NS5 | 2317 | 0.0140 | | |
| 1292.19 | 10 | KPTLHGPTPI | HIV | NS3 | 1614 | 0.2600 | | |
| 1292.20 | 10 | APTLWARMII | HIV | NS5 | 2835 | 0.3900 | | |
| 1292.22 | 10 | LPRRGPRLGI | HIV | Core | 37 | 0.6700 | | |
| 1292.23 | 9 | SPGQRVEFI | HIV | NS5 | 2615 | 0.0140 | | |
| 1292.24 | 9 | LPGCSFSII | HIV | Core | 169 | 0.1500 | | |
| 1292.26 | 10 | SPGALVVGVI | HIV | NS4 | 1887 | 0.0220 | | |
| 1292.27 | 10 | TPLLYRLGAI | HIV | NS3 | 1621 | 0.0220 | | |
| 27.0136 | 9 | APAAPTPAA | p53 | | 76 | 0.3000 | | |
| 27.0262 | 10 | APAPAAPTPA | p53 | | 74 | 0.0190 | | |
| 27.0264 | 10 | APSWPLSSSV | p53 | | 88 | 0.0230 | | |
| 28.0418 | 9 | FPWDILFPA | HDV | | 194 | 0.0200 | | |
| 34.0074 | 8 | IPWQRLLL | CEA | | 13 | 0.1100 | | |
| 34.0075 | 8 | RPGVNLSL | CEA | | 428 | 0.0720 | | |
| 34.0081 | 8 | SPGGLREL | HER2/neu | | | 133 | 0.0550 | |
| 34.0084 | 8 | WPDSLPDL | HER2/neu | | | 415 | 0.0200 | |
| 34.0085 | 8 | IPVAIKVL | HER2/neu | | | 748 | 0.0120 | |
| 34.0086 | 8 | SPYVSRLL | HER2/neu | | | 779 | 0.0440 | |
| 34.0087 | 8 | VPIKWMAL | HER2/neu | | | 884 | 1.4000 | |
| 34.0089 | 8 | SPKANKEI | HER2/neu | | | 760 | 0.0580 | |
| 34.0095 | 8 | RPRFRELV | HER2/neu | | | 966 | 0.0410 | |
| 34.0099 | 8 | SPGKNGVV | HER2/neu | | | 1174 | 0.0230 | |
| 34.0110 | 8 | VPISHLYI | MAGE2 | | 170 | 0.0170 | | |
| 34.0111 | 8 | MPKTGLLI | MAGE2 | | 196 | 0.0190 | | |
| 34.0117 | 8 | MPKAGLLI | MAGE3 | | 196 | 0.1300 | | |
| 34.0121 | 8 | APAPSWPL | p53 | | 86 | 0.0540 | | |
| 34.0178 | 9 | GPLPAARPI | HER2/neu | | | 1155 | 0.0550 | |
| 34.0180 | 9 | LPTNASLSI | HER2/neu | | | 65 | 0.0110 | |
| 34.0181 | 9 | SPAFDNLYI | HER2/neu | | | 1214 | 0.0190 | |
| 34.0182 | 9 | SPKANKEII | HER2/neu | | | 760 | 0.0150 | |
| 34.0183 | 9 | SPLTSIISI | HER2/neu | | | 649 | 0.0640 | |
| 34.0184 | 9 | SPREGPLPI | HER2/neu | | | 1151 | 0.1200 | |
| 34.0187 | 9 | GPHISYPPI | MAGE3 | | 296 | 0.0220 | | |
| 34.0190 | 9 | RPILTIITI | p53 | | 249 | 0.0460 | | |
| 34.0192 | 9 | SPQPKKKPI | p53 | | 315 | 0.0480 | | |
| 34.0260 | 10 | GPASPLDSTF | HER2/neu | | | 995 | 0.0110 | |
| 34.0265 | 10 | SPREGPLPAI | HER2/neu | | | 1151 | 0.0660 | |
| 34.0268 | 10 | VPISHLYILI | MAGE2 | | 170 | 0.0150 | | |
| 34.0271 | 10 | MPKAGLLIII | MAGE3 | | 196 | 0.0170 | | |
| 34.0273 | 10 | APAPAPSWPI | p53 | | 84 | 0.1300 | | |
| 34.0361 | 11 | SPLDSTFYRSL | HER2/neu | | | 998 | 0.0640 | |
| 34.0362 | 11 | LPAARPAGATL | HER2/neu | | | 1157 | 0.0140 | |
| 34.0365 | 11 | KPYDGIPAREI | HER2/neu | | | 921 | 0.0430 | |
| 34.0368 | 11 | SPLTSIISAVV | HER2/neu | | | 649 | 0.0250 | |
| 34.0374 | 11 | CPSGVKPDLSY | HER2/neu | | | 600 | 0.0300 | |
| 34.0382 | 11 | GPRALIETSYV | MAGE2 | | 274 | 0.1300 | | |
| 34.0387 | 11 | MPKAGLLIIVL | MAGE3 | | 196 | 0.0280 | | |
| 34.0389 | 11 | GPRALVETSYV | MAGE3 | | 274 | 0.1900 | | |
| 34.0390 | 11 | APRMPEAAPPV | p53 | | 63 | 0.4500 | | |
| 34.0397 | 11 | SPALNKMFBQI | p53 | | 127 | 0.1800 | | |

## Claims

1. A vaccine composition comprising an immunogenic peptide having a B7-like supermotif, which immunogenic peptide has SEQ ID No: 13 and is about 9 to 10 residues in length.

2. The vaccine composition of claim 1, further comprising a pharmaceutically acceptable excipient.

3. The vaccine composition of claim 1, wherein the peptide is admixed or linked to a second molecule.

4. The vaccine composition of claim 1, further comprising a liposome.

5. The vaccine composition of claim 1, wherein the peptide is complexed with an HLA molecule sharing a B7-like specificity that is present on an antigen-presenting cell.

6. A vaccine composition according to claim 1 for inducing a cytotoxic T cell response against hepatitis C in a patient.

7. An immunogenic peptide having a B7-like supermotif, which immunogenic peptide has SEQ ID NO: 13 for use in a method of medical treatment.

8. An immunogenic peptide having a B7-like supermotif, which immunogenic peptide has SEQ ID NO: 13 for inducing a cytotoxic T cell response against hepatitis C in a patient.

## Patentansprüche

1. Impfstoffzusammensetzung, die ein immunogenes Peptid enthält, mit einem B7-ähnlichen Supermotiv, wobei das immunogene Peptid die SEQ ID Nr. 13 hat und etwa 9 bis 10 (Aminosäuren-) Reste lang ist.

2. Impfstoffzusammensetzung nach Anspruch 1, die des weiteren einen pharmazeutisch verträglichen Hilfsstoff enthält.

3. Impfstoffzusammensetzung nach Anspruch 1, **dadurch gekennzeichnet, daß** das Peptid mit einem zweiten Molekül gemischt oder verlinkt ist.

4. Impfstoffzusammensetzung nach Anspruch 1, die des weiteren ein Liposom enthält.

5. Impfstoffzusammensetzung nach Anspruch 1, **dadurch gekennzeichnet, daß** das Peptid mit einem HLA-Molekül komplexiert ist, das eine B7-ähnliche Spezifizität teilt, die sich auf einer Antigen präsentierenden Zelle befindet.

6. Impfstoffzusammensetzung nach Anspruch 1, zur Induktion einer zytotoxischen T-Zell-Antwort gegen Hepatitis C bei einem Patienten.

7. Immunogenes Peptid, das ein B7-ähnliches Supermotiv aufweist, wobei das immunogene Peptid die SEQ ID Nr. 13 hat, zur Verwendung in einem Verfahren zur medizinischen Behandlung.

8. Immunogenes Peptid, das ein B7-ähnliches Supermotiv aufweist, wobei das immunogene Peptid die SEQ ID Nr. 13 hat, zur Induktion einer zytotoxischen T-Zell-Antwort gegen Hepatitis C bei einem Patienten.

## Revendications

1. Composition de vaccin comprenant un peptide immunogène ayant un supermotif de type B7, lequel peptide immunogène possède SEQ ID NO : 13 et a une longueur d'environ 9 à 10 résidus.

2. Composition de vaccin selon la revendication 1, comprenant en outre un excipient pharmaceutiquement acceptable.

3. Composition de vaccin selon la revendication 1, dans laquelle le peptide est mélangé ou lié à une seconde molécule.

4. Composition de vaccin selon la revendication 1, comprenant en outre un liposome.

5. Composition de vaccin selon la revendication 1, dans laquelle le peptide est complexé avec une molécule HLA partageant une spécificité de type B7 qui est présente sur une cellule présentant l'antigène.

6. Composition de vaccin selon la revendication 1, pour induire une réponse des lymphocytes T cytotoxiques contre l'hépatite C chez un patient.

7. Peptide immunogène comportant un supermotif de type B7, lequel peptide immunogène possède SEQ ID NO : 13, à utiliser dans un procédé de traitement médical.

8. Peptide immunogène comportant un supermotif de type B7, lequel peptide immunogène possède SEQ ID NO : 13 pour induire une réponse des lymphocytes T cytotoxiques contre l'hépatite C chez un patient.
